Europäisches Patentamt

European Patent Office

Office européen des brevets

(19)

(11) Numéro de publication : **0 434 563 A1**

# (12) DEMANDE DE BREVET EUROPEEN

(21) Numéro de dépôt : **90403693.6**

(51) Int. Cl.⁵ : **C12N 9/08**

(22) Date de dépôt : **20.12.90**

(30) Priorité : **21.12.89 FR 8917001**

(43) Date de publication de la demande :
**26.06.91 Bulletin 91/26**

(84) Etats contractants désignés :
**AT BE CH DE DK ES FR GB GR IT LI LU NL SE**

(71) Demandeur : **SOCIETE NATIONALE ELF
AQUITAINE
Tour Elf, 2, Place de la Coupole, La Défense 6
F-92400 Courbevoie (FR)**

(72) Inventeur : **Bono, Françoise
42, rue de Varsovie
F-31300 Toulouse (FR)**
Inventeur : **Cellard, Hervé
6, rue de Naurouze
F-31750 Escalquens (FR)**
Inventeur : **Le Bouteiller, Christine
23, rue de L'Amandier
F-31100 Toulouse (FR)**
Inventeur : **Seris, Jean-Louis
Lotissement Parenche, Chemin Vignats
F-64110 Jurançon (FR)**

(74) Mandataire : **Ohresser, François et al
Société Nationale Elf Aquitaine Division
Proprieté Industrielle Tour Elf
F-92078 Paris La Défense Cédex 45 (FR)**

(54) **Procédé de production de peroxydases MN-dépendantes.**

(57)  Procédé de production de l'enzyme Peroxydase Mn-Dépendante par culture du champignon Phanerochaete Chrysosporium.
Le procédé se caractérise par l'utilisation d'un milieu de culture contenant de la polyvinylpyrrolidone.

EP 0 434 563 A1

# PROCEDE DE PRODUCTION DE PEROXYDASES MN-DEPENDANTES

L'invention a pour objet un procédé amélioré de production de l'enzyme Peroxydase Mn-Dépendante par culture du champignon **Phanerochaete** Chrysosporium.

La lignine est avec la cellulose, le composé le plus abondant sur terre. Elle constitue en effet 17% à 33% du bois. Par ailleurs, ce polymère aromatique pose un sérieux problème à l'industrie papetière puisqu'il est à l'origine de la coloration jaune de la pâte à papier.

Actuellement, de nombreux procédés chimiques de blanchiment permettent la quasi élimination de la lignine, mais entraînent parallèlement des rejets polluants constitués par des composés chlorés qui pourraient être prochainement interdits par diverses législations.

En raison de cette politique de protection de l'environnement, l'industrie papetière s'est donc intéressée au blanchiment biologique de la pâte à papier par l'utilisation de micro-organismes dits ligninolytiques. De nombreux champignons ainsi que quelques bactéries sont capables de dégrader le bois. Cependant seuls les champignons classés parmi les pourritures blanches dégradent la lignine jusqu'au stade ultime. (Kirk.T.K. Farrel, R (1987). Enzymatic combustion. the microbial degradation of lignin ; Annual Review Microbiology, 41, 465. 505.

Parmi les nombreuses souches testées, **Phanerochaete** Chrysosporium semble présenter les meilleures activités enzymatiques.

Ainsi deux groupes d'enzymes ont été identifiées comme étant en partie responsables de la dégradation de la lignine : les lignines peroxydases et les peroxydases Mn-Dépendantes. Celles-ci sont décrites dans la littérature.

La demande de brevet européen 188931 concerne des champignons produisant des enzymes ligninases. Par ailleurs, l'enzyme peroxydase Mn(II) dépendante issue de Phanerochaete chrysosporium est décrite par J. K. GLENN et al dans "Archives of Biochemistry and Biophysics" vol. 242 n°2 November pp 329.341, 1985.

Le but de l'invention consiste donc à augmenter la production de l'une de ces enzymes, les Peroxydases Mn-Dépendantes, ci-après désignées MnPo en agissant sur certains paramètres intervenant dans la fermentation du champignon.

Les Péroxydases Mn-Dépendantes (MnPo) sont des enzymes extracellulaires catalysant l'oxydation Mn-Dépendante de plusieurs phénols et colorants.

D'un poids moléculaire de 46 kd, elles oxydent les ions $Mn^{2+}$ en ions $Mn^{3+}$ qui sont les véritables agents d'oxydation des composés cités ci-dessus.

Il a été découvert avec surprise que l'addition au milieu d'un polymère artificiel, la polyvinyl pyrrolidone, connue pour ses propriétés complexantes et antiradicalaires notamment permettait d'augmenter considérablement l'activité MnPo du milieu.

La présente invention a pour objet un procédé de production des enzymes Peroxydases Mn-Dépendantes par culture du champignon **Phanerochaete** Chrysosporium dans un milieu contenant au moins une source de carbone métabolisable par le champignon dans des conditions classiques de culture caractérisé en ce qu'on ajoute au milieu de la polyvinyl pyrrolidone.

Les conditions générales de culture sont les suivantes. Le procédé de l'invention sera mis en oeuvre avec un substrat de croissance carboné métabolisable par le champignon. Ce substrat sera de préférence un composé porteur de plusieurs fonctions alcools, tel que par exemple le glucose, facilement et économiquement accessible, ou le glycérol. Ce dernier substrat sera particulièrement efficace lorsqu'il sera complémenté par une deuxième source de carbone constitué par un sel minéral d'un acide organique, par exemple un acétate d'un métal alcalin tel que l'acétate de sodium. Cette deuxième source de carbone sera généralement ajoutée en une fraction molaire représentant 1 à 20% et de préférence 5 à 15% du substrat principal.

Le milieu comportera également au moins une source d'azote et tous les oligo-éléments (sels) nécessaires à la croissance. Comme source d'azote, on pourra utiliser les sels d'ammonium : sels minéraux ou sels organiques, les aminoacides : acide aspartique, acide glutamique (également, source de carbone), urée, des extraits de malt...

A titre d'exemple de sels minéraux, oligo-éléments utilisables dans le milieu de culture, on peut citer les sels de magnésium $(MgSO_4)$, de manganèse $(MnSO_4)$, de fer $(FeSO_4)$, de potassium $(KH_2PO_4)$, de calcium $(CaCl_2)$, de sodium (NaCl), etc..

On peut également ajouter une substance permettant une meilleure croissance du champignon comme des vitamines si nécessaires ; la thiamine est particulièrement bien adaptée à ce type de micro-organisme.

Le procédé de culture du champignon est mis en oeuvre classiquement à un pH légèrement acide généralement compris entre 4 et 6. Le procédé de culture est mis en oeuvre en aérobie à des températures habituelles pour ce type de procédé généralement comprises entre 25 et 39°C.

Enfin, il sera avantageux, pour obtenir une bonne productivité et une bonne reproductibilité du résultat d'agiter le milieu de culture par un moyen convenable, par exemple agitation rotative ou alternative.

La caractéristique principale du procédé de l'invention réside dans l'addition au milieu de culture d'un polymère synthétique la polyvinyl pyrrolidone (PVP).

La masse moléculaire moyenne de la PVP sera généralement comprise entre 1000 et 1000000 daltons. On pourra utiliser les polymères habituellement mis sur le marché qui ont des masses moléculaires moyennes de 2500, 10000, 40000 et 750000 daltons. La limite supérieure de cette masse est simplement liée à la solubilité du polymère dans le milieu. La quantité de PVP ajoutée au milieu sera supérieure à 0,5 g/l. Il n'y a pas de limite supérieure à cette quantité sinon celle qui est déterminée par la solubilisation de la PVP dans le milieu et par la viscosité du milieu qu'entraîne son addition. Cette quantité sera généralement comprise entre 2,5 et 10 g/l et de préférence entre 5 et 7,5 g/l.

Les performances du procédé de production de l'enzyme MnPo pourront être optimisées et c'est une autre caractéristique du procédé, si l'on utilise un milieu de culture ayant des teneurs élevées en certains sels classiques, c'est-à-dire utilisés dans les milieux de culture standard, et/ou comprenant du chlorure ferrique $FeCl_3$.

Il sera particulièrement intéressant d'ajouter au milieu une quantité importante de $MnSO_4$, $H_2O$, c'est-à-dire que sa concentration au sein du milieu de culture sera généralement comprise entre 10 et 40 mg/l, alors que dans les milieux standards elle est généralement de l'ordre de 10 fois moindre.

Il sera également intéressant d'ajouter au milieu du $FeSO_4$, 7 $H_2O$ à une quantité telle que sa concentration soit comprise entre 2 et 10 mg/l, au lieu de 0,5 mg/l environ pour les cultures standards.

Enfin, l'addition de $FeCl_3$ à une concentration comprise entre 0,1 et 1,5 mg/l, pourra avoir un effet favorable sur l'activité enzymatique MnPo et sa production.

Une autre caractéristique du procédé réside dans le choix de la température de culture qui sera de préférence comprise entre 30 et 35°C, domaine optimal pour la croissance du champignon et l'excrétion de l'enzyme MnPo.

Un autre paramètre du procédé de culture réside dans l'utilisation d'une atmosphère contrôlée en oxygène. La pression partielle en oxygène sera généralement comprise entre 20 et 60% et de préférence entre 20 et 40%, la culture s'effectuant classiquement à la pression atmosphérique. Ceci va à l'encontre de l'enseignement antérieur dans la mesure où tous les travaux menés jusqu'à maintenant étaient orientés vers une très forte concentration en oxygène proche de 100%.

Le procédé de l'invention sera mieux compris à la lecture des exemples ci-après donnés à titre d'illustration non limitative.

Les conditions de culture du champignon <u>Phaneroachaete chrysosporium</u> (souche ATCC 24725) utilisées pour ces exemples sont décrites ci-après :

Milieu de culture (standard, concentrations exprimées en poids par litre de milieu final) :

- glycérol 4.34 g
- tartrate d'ammonium 2.125 g
- $KH_2PO_4$ 1.74 g
- $MgSO_4$, 7 $H_2O$ 0.44 g
- $CaCl_2$, 2 $H_2O$ 0.087 g
- chlorhydrate de thiamine 0.00087 g
- alcool vératrylique 0.0583 g
- tampon acétate 0.27 g (acide acétique ajusté à pH = 4.5 avec NaOH)
- $MgSO_4$, 7 $H_2O$ 0.01823 g
- $MnSO_4$, $H_2O$ 0.00303 g
- NaCl 0.0061 g
- $FeSO_4$, 7 $H_2O$ 0.00061 g
- $CoCl_2$ 0.00061 g
- $ZnSO_4$, 7 $H_2$ 0.00061 g
- $CaCl_2$, 2 $H_2O$ 0.00061 g
- $CuSO_4$, 5 $H_2O$ 0.000061 g
- $AlK(SO_4)_2$, 12 $H_2O$ 0.000061 g
- $H_3BO_3$ 0.000061 g
- $Na_2MoO_4$, 2 $H_2O$ 0.000061 g

Conditions de culture :

Le champignon est cultivé dans des erlenmeyers de 500 ml contenant 100 ml de milieu de culture. Les fioles sont inoculées avec 2 ml d'une suspension de spores à 5-6.10⁶ spores/ml (comptage à la cellule de Malassez, suspension réalisée dans de l'eau milliQ supplémentée avec 0.1% de tween 80). Les fioles restent bouchées pendant toute la durée de la culture, c'est-à-dire qu'il n'y a pas d'échanges gazeux avec l'extérieur sauf pendant

3

15 à 20 min. après l'inoculation, puis le quatrième jour de la croissance et les jours suivants où l'atmosphère de la fiole est renouvelée avec de l'air comprimé. L'incubation a lieu dans des conditions agitées dans un shaker rotatif à 32.5°C et à 140 tours/min.

Pour contrôler l'effet des divers composés ajoutés au milieu de culture, on mesure l'activité Mn-Peroxydase exprimés en unités enzymatiques par litre de solution (1 UE = 1 nmole de substrat transformé par minute) au 8ème jour de culture.

On mesure successivement l'activité lignine-peroxydase LiPo, puis l'activité Mn Peroxydase.

L'activité Lignine-Péroxydase est mesurée dans le surnageant de culture par la détermination du taux d'oxydation de l'alcool vératrylique en aldéhyde à 37°C en suivant l'augmentation de la densité optique à la longueur d'onde de 310 nm.

Le mélange réactionnel comprend : 1.88 ml de tampon lactate 0.1 M, pH = 3, 1 ml de surnageant et 100 μl d'alcool vératrylique. Après homogénéisation et stabilisation à 37°C, la réaction est induite par l'addition de 20 μl d'eau oxygénée.

Calcul : L'activité LiPo est exprimée en unités enzymatiques par litre de solution (1 UE = 1 nmole de substrat transformée par minute), le coefficient d'extinction molaire = 9300 mole.$l^{-1}$ cm$^{-1}$, volume réactionnel = 3 ml.

L'activité Mn-Péroxydase est mesurée dans le surnageant de culture par la détermination de la disparition de la Vanilline Acétone à 37°C. Cette disparition s'explique par l'oxydation des ions Mn$^{2+}$ en ions Mn$^{3+}$ qui transforment alors la Vanilline Acétone. Cela se traduit par une diminution de la densité optique à 336 nm.

Le mélange réactionnel comporte : 1.79 ml de tampon lactate 0.1 M, pH = 4.5, 1 ml de surnageant de culture, 100 μl de Vanilline Acétone (0.6 g/l). Après homogénéisation et stabilisation à 37°C, l'addition de 10 μl d'eau oxygénée provoque la dégradation du substrat par les Lignine-Péroxydase (LiPo) (1ère pente négative). Ensuite, l'addition de 100 l de MnSO$_4$, H$_2$O (3 mM) fait apparaître une seconde pente négative correspondant à l'action cumulée des LiPo et MnPo sur le substrat. La différence entre les deux pentes va donc traduire l'activité des MnPo (exprimée également en UE/l).

Le substrat glycérol est dosé par l'utilisation du kit enzymatique Boehringer (124.270). A partir du glycérol, une série de trois réactions catalysées chacune par trois enzymes différentes, aboutit à la formation de NAD$^+$ dont la quantité est stoechiométrique à celle du glycérol. Le NADH disparu est alors dosé à la longueur d'onde de 365 nm.

Exemple 1 :

L'exemple 1 illustre une culture de référence réalisée avec le milieu standard décrit ci-dessus sans addition de PVP. L'activité enzymatique obtenue était : LiPo 561 et MnPo 177 (UE/l).

Exemples 2 à 11 :

Les différents exemples comparatifs (2 à 11) suivants ont été réalisés au moyen du milieu de culture décrit ci-dessus modifié comme suit : 29.6 mg/l de MnSO$_4$ (au lieu de 3.03 mg/l), 5.3 mg/l de FeSO-4, 7 H$_2$O (au lieu de 0.61 mg/l) ; 0.6 mg/l de FeCl$_3$.

Dans ces exemples, on a fait varier la masse moléculaire de la PVP ; celle-ci étant ajoutée à la concentration de 5 g/l. Les exemples 2 et 3 servent de témoin sans PVP.

Les résultats obtenus, exprimés en activités enzymatiques LiPo MnPo-mesurées après 8 jours de culture sont résumés dans le tableau 1 ci-après :

4

EP 0 434 563 A1

**Tableau 1**

| Ex | PVP (MM:daltons) | ACTIVITE LiPo (UE/l) | Activité MnPo (UE/l) |
|---|---|---|---|
| 2 | – | 710 | 393 |
| 3 | – | 516 | 361 |
| 4 | 2 500 | 807 | 755 |
| 5 | 2 500 | 1 032 | 1 337 |
| 6 | 10 000 | 936 | 1 593 |
| 7 | 10 000 | 936 | 1 115 |

| 8 | 25 000 | 1 100 | 1 443 |
|---|---|---|---|
| 9 | 25 000 | 939 | 1 377 |
| 10 | 40 000 | 903 | 1 213 |
| 11 | 40 000 | 742 | 853 |

Exemples 12 et 13 :

Les exemples 12 et 13 illustrent l'influence de la concentration en PVP sur l'activité enzymatique MnPo ; les expériences ont été réalisées avec un milieu analogue à celui utilisé dans les exemples 2 à 11, avec cependant une teneur en $FeCl_3$ de 0.3 mg/l et une teneur en $FeSO_4$, 7 $H_2O$ de 0,61 mg/l, en faisant varier les concentrations en PVP de PM 40000. Exemple 12 : 2.5 g/l, exemple 13 : 7.5 g/l ; les activités enzymatiques obtenues étaient les suivantes :

Exemple 12 : LiPo 939, MnPo : 1610 (UE/1)

Exemple 13 : LiPo 939, MnPo : 1862 (UE/1)

Ces différentes expériences illustrent l'influence positive de l'addition de PVP au milieu de culture sur l'activité MnPo, et à un degré moindre sur l'activité LiPo.

En effet, si l'on compare les résultats obtenus à l'exemple 1 pour une culture de référence qui sont de l'ordre de 200 UE/1 au maximum et ceux obtenus avec de la PVP à différentes concentrations 2.5, 5, 7.5 g/l, on observe une augmentation considérable de l'activité MnPo qui passe en moyenne à un niveau de 1500-1600 UE/1.

La comparaison de l'exemple 1 et de l'exemple 2 et 3 illustre l'influence favorable de l'addition au milieu de $FeCl_3$ d'une part, et de quantités importantes de $MnSO_4$, $H_2O$ qui double sensiblement l'activité enzymatique de MnPo en l'absence de la PVP.

Enfin, il est intéressant de constater que quelle que soit la masse moléculaire de la PVP utilisée, l'effet favorable sur les activités enzymatiques est sensiblement le même. La même observation peut être faite pour la quantité de PVP ajoutée dès lors que celle-ci est substantielle. (> 2,5 g/l).

5

**Revendications**

1. Procédé de production d'enzymes Peroxydases Mn-Dépendantes par culture du champignon <u>Phanero-chaete Chrysosporium</u> dans un milieu contenant au moins une source de carbone métobolisable et tous les éléments nécessaires à la croissance du champignon caractérisé en ce qu'on ajoute au milieu de culture de la polyvinylpyrrolidone.

2. Procédé selon la revendication 1 caractérisé en ce que la quantité de polyvinylpyrrolidone ajoutée est telle que sa concentration dans le milieu est comprise entre 0,5 g/l et 10 g/l.

3. Procédé selon l'une des revendications 1 et 2 caractérisé en ce que le milieu de culture contient de 5 à 40 mg/l de $MnSO_4, H_2O$.

4. Procédé selon l'une des revendications précédentes caractérisé en ce que le milieu de culture contient de 0,1 à 10 mg/l de $FeCl_3$.

5. Procédé selon l'une des revendications précédentes caractérisé en ce qu'il est mis en oeuvre à une température comprise entre 30 et 35°C.

6. Procédé selon l'une des revendications précédentes caractérisé en ce qu'il est mis en oeuvre à une pression partielle d'oxygène comprise entre 20 et 60%.

Office européen
des brevets

Numero de la demande

# RAPPORT DE RECHERCHE EUROPEENNE

EP  90 40 3693

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-4 743 550  (P.K. ANANTHAPADMANABHAN et al.) * abrégé; colonne 2; pages 49-63; colonne 4, lignes 53-68; revendications * | 1 | C 12 N   9/08 |
| D,Y | EP-A-0 188 931  (INSTITUT NATIONAL DE LA RECHERCHE AGRONOMIQUE) * document en entier * | 1 | |
| A | BIOLOGICAL ABSTRACTS DATABANK abrégé no. 87036692; A.F. HSU et al.: "Evaluation of several methods of estimation of the total activity of potato polyphenol oxidase" & Journal of Food Science 1988, vol. 53, no. 6, pages 1743-1745 | 1 | |
| Y | CHEMICAL ABSTRACTS vol. 95, no. 11, 14 septembre 1981, page 239, abrégé no. 92929x, Columbus, Ohio, US; F.J. CASTILLO et al.: "Masking and uncovering isoperoxidases from Pelargonium" & C. R. Seances Acad. Sci., Ser. 3 1981, vol. 292, no. 2, pages 259-262 | 1 | **DOMAINES TECHNIQUES RECHERCHES (Int. Cl.5)**<br><br>C 12 N   9/00<br>C 12 N   1/14 |
| D,Y | ARCHIVES OF BIOCHEMISTRY AND BIOPHYSICS vol. 242, no. 2, 1 novembre 1985, pages 329-341, Orlando, FL, US; J.K. GLENN et al.: "Purification and Characterization of an Extracellular Mn(II)-Dependent Peroxidase from the Lignin-Degrading Basidiomycete, Phanerochaete chrysosporium" * document en entier *                  -/- | 1 | |

Le présent rapport a été établi pour toutes les revendications

| Lieu de la recherche | Date d'achèvement de la recherche | Examinateur |
|---|---|---|
| BERLIN | 19-02-1991 | JULIA P. |

EPO FORM 1503 03.82 (P0402)

EP 0 434 563 A1

Page    2

Office européen
des brevets

RAPPORT DE RECHERCHE EUROPEENNE

Numero de la demande

EP  90 40 3693

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 303 062  (BIOTEST AG) <br> * abrégé; page 3, colonne 3, lignes 3-7; colonne 4, exemples 4,7,8; revendication 1 * | 1 | |
| D,A | ANN. REV. MICROBIOL. vol. 41, 1987, pages 465-505, Palo Alto, CA, US; T.K. KIRK et al.: "Exzymatic "Combustion": the Microbial Degradation of Lignin" <br> * pages 473,482,483 * | 1 | |
| A | FEMS MICROBIOLOGY LETTERS vol. 29, 1985, pages 37-41, Amsterdam, NL; A. PASZCZYNSKI et al.: "Enzymatic activities of an extracellular, manganese-dependent peroxidase from Phanerochaete chrysosporium" <br> * document entier * | 1 | |
| A | EP-A-0 223 221  (BOEHRINGER MANNHEIM GMBH) <br> * document entier * | 1 | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| BERLIN | 19-02-1991 | JULIA P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)